# EUROPÄISCHE PATENTANMELDUNG

(11) **EP 2 143 405 A1**
(43) Veröffentlichungstag der Anmeldung: **13.01.2010**
(21) Anmeldenummer: 09162545.9
(22) Anmeldetag: 12.06.2009
(51) Int. Cl.: A61F 2/82

(54) **Stent mit biodegradierbaren Stentstreben und Wirkstoffdepots**

(30) Priorität: 11.07.2008 DE 102008040356
(71) Anmelder: Biotronik VI Patent AG, 6341 Baar (CH)
(72) Erfinder: Klocke, Björn, 8006, Zürich (CH)
(74) Vertreter: Lindner-Vogt, Karin L.

(57) **Zusammenfassung**

Ein Stent umfasst Stentstreben (1) aus einem biodegradierbaren Polymermaterial, Wirkstoffdepots (4) mit mindestens einem Wirkstoff in den Stentstreben (1), mindestens einen die Abbaufolgen der Stentstreben (1) therapierenden Wirkstoff in den Wirkstoffdepots (4), und eine zeitlich veränderliche Umhüllung der Wirkstoffdepots (4) derart, dass die Freisetzung des Wirkstoffs aus den Wirkstoffdepots (4) auf den Massenabbau des biodegradierbaren Polymermaterials der Stentstreben (1) zeitlich abgestimmt erfolgt.

## Beschreibung

Die Erfindung betrifft einen Stent umfassend Stentstreben aus einem biodegradierbaren Polymermaterial und Wirkstoffdepots mit mindestens einem Wirkstoff in diesen Stentstreben.

Der aktuelle Stand der Technik bei sogenannten Polymerstents aus einem biodegradierbaren Material sieht einphasige Stentgrundkörper vor, die gegebenenfalls an ihrer Oberfläche mit einer dünnen Wirkstoff abgebenden Schicht versehen sind. Letztere kann beispielsweise ein anti-proliferativ wirkendes Pharmazeutikum enthalten. Auch ist es bekannt, Wirkstoffdepots innerhalb von Stentstreben anzulegen, so beispielsweise aus der US 2006/0224234 A1. Der dort offenbarte Stent soll jedoch ausdrücklich frei von Polymermaterial sein.

Aus der US 2004/0204750 A1 ist ein Wirkstoff eluierender Stent für die kontrollierte Wirkstoffabgabe bekannt, der auf Polymerbasis gefertigt ist. Es handelt sich dabei nicht um biologisch abbaubare Stents. In den Stentstreben sind Wirkstoffdepots vorgesehen, die beispielsweise in Durchbrüchen angelegt und durch eine über die Stentstreben durchgehende Sperrschicht in ihrem Eluierungsverhalten definierbar sind. Eine Abstimmung auf das Degradationsverhalten der Stentstreben ist bei diesem Stand der Technik nicht vorgesehen.

Schließlich offenbart die US 2004/0220660 A1 einen bioresorbierbaren Stent, bei dem in Durchbrüchen der Stentstruktur wiederum Wirkstoffdepots angelegt sind. Diese sind nach innen zum Gefäßlumen hin durch eine Sperrschicht verschlossen, zur Gefäßwand hin sind sie offen, so dass aus dem Wirkstoffdepot die Wirkstoffe austreten können. Auch hier findet keine Korrelation zwischen dem Degradationsverhalten des bioresorbierbaren Stents und dem Austrittsverhalten der Wirkstoffe aus dem Depot statt.

Aktuelle Forschungsarbeiten haben nun im Zusammenhang mit bioresorbierbaren Stents aus einem biodegradierbaren Polymermaterial ergeben, dass sich die Polymere in vivo (und insbesondere human bei älteren Patienten) langsamer abbauen als dies die bislang vorherrschende Forschungshypothese war. Kurzkettige Polymere, wie sie während des Abbauprozesses anfallen, halten sich trotz Verlust der mechanischen Stabilität des Stents über längere Zeit, oft über Jahre, im Gewebe und verkürzen sich offensichtlich nur sehr langsam hin zu Monomeren, die dann durch das umliegende Körpergewebe abtransportiert werden können. Diese massive Formierung kurzkettiger Polymerbereiche im Gewebe bringt ein erhebliches Risiko mit sich, so dass zu einem bestimmten Zeitpunkt starke Inflammationen auftreten, die in der Literatur unter dem Stichwort "Tissue overload" behandelt werden. Teilweise kommt es auch zum unkontrollierten Abtransport von Polymerbruchstücken durch Makrophagen. Beide Effekte können zu ungewünscht hoher Inflammation führen, die sich negativ auf die Heilung des Gewebes nach der Stentimplantation auswirkt, und im schlimmsten Fall kann Polymermaterial in vergleichsweise großem Umfang in die Blutbahn gelangt und dort Thrombosen hervorrufen.

Zusammenfassend besteht also bei biologisch abbaubaren Polymerstents aus den üblichen Materialien, wie Polyestern, die über den Citrat-Zyklus abgebaut werden, die Gefahr, dass trotz kontinuierlicher Verkürzung der Polymerkette der eigentliche Massenabbau erst mit einem sogenannten "Bulk release" - also einer massiven Materialfreigabe - stattfindet, bei dem die Toleranzschwelle des umliegenden Gewebes für die Degradationsprodukte, insbesondere für Säuren überschritten werden kann. Dies kann zu einer übermäßigen Entzündungsreaktion und ggf. auch zu Spätthrombosen führen.

Davon ausgehend liegt der Erfindung die Aufgabe zugrunde, einen Stent aus biodegradierbarem Polymermaterial so zu verbessern, dass während des Abbaus des Stents auch späte Entzündungsreaktionen im Gewebe beherrschbar bleiben und es nicht zu Gewebezerstörung mit folgenden Thrombosen kommt.

Diese Aufgabe wird durch einen Stent mit den Merkmalen des Anspruch 1 gelöst, wonach vorgesehen sind:
- mindestens ein die Abbaufolgen der Stentstreben therapierender Wirkstoff in den Wirkstoffdepots, und
- eine zeitlich veränderliche Umhüllung der Wirkstoffdepots derart, dass die Freisetzung des Wirkstoffs aus den Wirkstoffdepots auf den Massenabbau des biodegradierbaren Polymermaterials der Stentstreben zeitlich abgestimmt erfolgt.

Aufgrund dieser Auslegung der Umhüllung der Wirkstoffdepots wird der für den oben angegebenen therapeutischen Zweck zu eluierende Wirkstoff genau dann frei, wenn die Degradation und insbesondere der problematische Massenabbau des Grundkörpers des Stents stattfindet. Damit kann den negativen Folgen dieses Ereignisses mithilfe des Wirkstoffes zielgenau entgegengewirkt werden.

Bevorzugte Ausführungsformen der Erfindung ergeben sich aus den abhängigen Ansprüchen. Weitere Merkmale, Einzelheiten und Vorteile in diesem Zusammenhang ergeben sich aus der nachfolgenden Beschreibung, in der Ausführungsbeispiele anhand der beigefügten Zeichnungen näher erläutert werden. Es zeigen:
- Fig. 1 bis 3: Querschnitte von Stentstreben in unterschiedlichen Ausführungsformen,
- Fig. 4: eine perspektivische Teilansicht einer Stentstrebe in einer weiteren Ausführung, und
- Fig. 5: einen Längsschnitt der Stentstrebe gemäß Fig. 4.

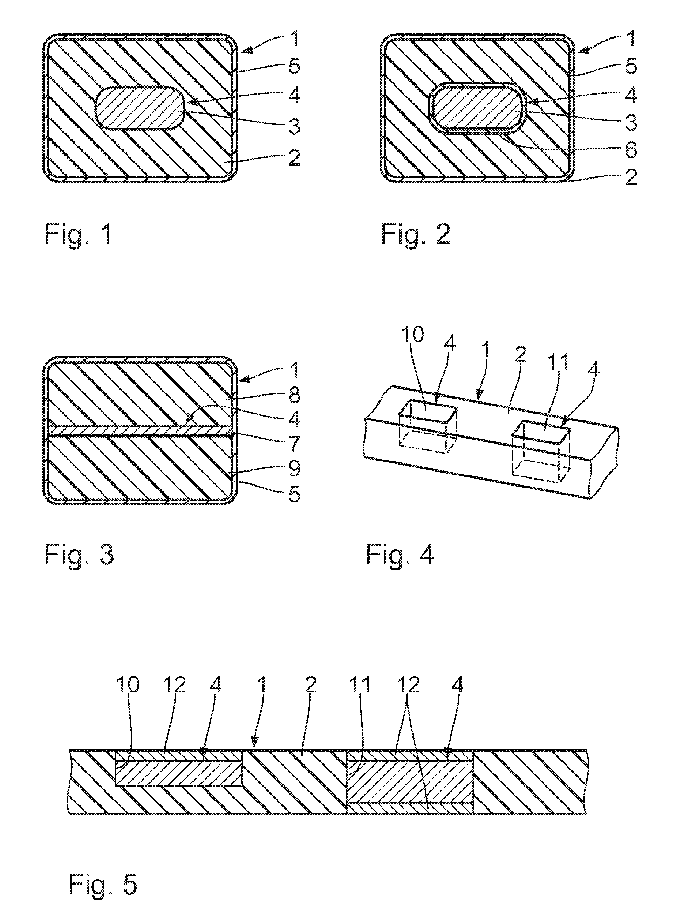

Die in Fig. 1 gezeigte Stentstrebe 1 besteht aus einem langgestreckten, im Querschnitt etwa rechteckigen Stentgrundkörper 2, der die mechanischen Eigenschaften des Stents im Wesentlichen bestimmt. Der Stentgrundkörper besteht aus einem biodegradierbaren Polymermaterial, wie beispielsweise PLLA.

In seinem Kern ist der Stentgrundkörper 2 mit einer Seele 3 in Form einer in Längsrichtung durchgehenden Röhre versehen, in der ein Wirkstoffdepot 4 angelegt ist. Damit umhüllt der Stentgrundkörper 2 komplett das Wirkstoffdepot 4. Letzteres umfasst einen oder mehrere Wirkstoffe, wie beispielsweise Inflammationshemmer, wie z.B. Paclitaxel oder Sirolimus und seine Derivate wie Biolimus, Everolimus, Deforolimus, Zotarolimus und andere, bzw. einen heilungsfördernden Wirkstoff oder antiphlogistische als auch antithrombotische Stoffe.

Als antiproliferative, antiinflammatorische und/oder antimykotische Wirkstoffe können beispielsweise die aus der folgenden Liste gewählt werden:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanole A,B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2w, Mitoxanthrone, Mycophenolatmofetil, c-myc-Antisense, [beta]-myc-Antisense, [beta]-Lapachon, Podophyllotoxin, Podophyllsäure-2-ethylhydrazid, Molgramostim (rhuGM-CSF), Peginterferon [alpha]-2b, Lanograstim (r-HuG-CSF), Macrogol, Selectin (Cytokinantagonist), Cytokininhibitoren, COX-2-Inhibitor, NFkB, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren wie Pentaerythrityltetranitrat und Syndnoeimine, S-Nitrosoderivate, Tamoxifen, Staurosporin, [beta]-Estradiol, [alpha]-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel und dessen Derivate wie 6-[alpha]-Hydroxy-Paclitaxel, Taxotere, Kohlensuboxids (MCS) und dessen macrocyclische Oligomere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, [beta]-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline wie Dicloxacillin, Oxacillin, Sulfonamide, Metronidazol, Enoxoparin, desulfatiertes und N-reacetyliertes Heparin (Hemoparin<(R)>), Gewebe-Plasminogen-Aktivator, Gpllb/Illa-Plättchenmembranrezeptor, Faktor Xa-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon [alpha], [beta] und [gamma], Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol,, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

Als antiproliferative Wirkstoffe werden bevorzugt Cytostatika, Makrolidantibiotika, und/oder Statine eingesetzt. Geeignete antiproliferative Wirkstoffe sind Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid Estramustin, Melphalan, Betulinsäure, Camptothecin, Lapachol, [beta]-Lapachon, Podophyllotoxin, Betulin, Tropfosfamid, Podophyllsäure-2-ethylhydrazid, Ifosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Mofebutazon, Acemetacin, Diclofenac, Lonazolac Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, [beta]-Sitosterin, Ademetionin, Myrtecain, Polidocanol, Nonivamid, Levomenthol, Benzocain, Aescin, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense Selectin (Cytokinantagonist) CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, Folsäure und Derivate, Vitamine der B-Reihe, Vitamin D-Derivate wie z.B. Calcipotriol und Tacalcitol, Thymosin [alpha]-1, Fumarsäure und ihre Derivate wie z.B. Dimethylfumarat, IL-1[beta]-Inhibitor, Colchicin, NO-Donoren wie Pentaerthrityltetranitrat und Syndnoeimine, S-Nitrosoderivate, Tamoxifen, Staurosporin, [beta]-Estradiol, [alpha]-Estradiol, Estron, Estriol, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate (6-[alpha]-Hydroxy-Paclitaxel, Baccatin, Taxotere u.a.), synthetisch hergestellte als auch aus nativen Quellen gewonnene macrocyclische Oligomere des Kohlensuboxids (MCS) und seine Derivate, Molgramostim (rhuGM-CSF), Peginterferon [alpha]-2b, Lanograstim (r-HuG-CSF), Filgrastim, Macrogol, Dacarbazin, Basiliximab, Daclizumab, Ellipticin, D-24851 (Calbiochem), Colcemid, Cytochalasin A-E, Indanocine, Nocadazole, S 100 Protein, PI-88, Melanocyte Stimulating Hormon ([alpha]-MSH), Bacitracin, Vitronectin-Rezeptor Antagonisten, Azelastin, Guanidylcyclase-Stimulator, Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1 genannt. Aus der Gruppe der Antibiotika finden des weiteren Cefadroxil, Cefazolin, Cefaclor, Cefotixin Tobramycin, Gentamycin Anwendung. Positiven Einfluss auf die postoperative Phase haben auch Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, Hemoparin<(R)> (desulfatiertes und N-reacetyliertes Heparin), Gewebe-Plasminogen-Aktivator, GpIIb/IIIa-Plättchenmembranrezeptor, Faktor Xa-Inhibitor, aktiviertes Protein C, Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Caspaseinhibitoren, Apoptoseinhibitoren, Apoptoseregulatoren wie p65, NF-kB und Bcl-xL-Antisense-Oligonukleotiden und Prostacyclin, Vapiprost, [alpha], [beta]- und [gamma]-Interferon, Histaminantagonisten, Serotoninblocker, Halofuginon, Nifedipin, Tocopherol, Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron. Weitere Wirkstoffe sind Steroide (Hydrocortison, Betamethason, Dexamethason), nichtsteroidale Substanzen (NSAIDS) wie Fenoporfen, Ibuprofen, Indomethacin, Naproxen, Phenylbutazon und andere. Antivirale Agentien wie Acyclovir, Ganciclovir und Zidovudin sind ebenfalls einsetzbar. Verschiedene Antimykotika finden Anwendung in diesem Bereich. Beispiele sind Clotrimazol, Flucytosin, Griseofulvin, Ketoconazol, Miconazol, Nystatin, Terbinafin. Antiprozoale Agentien wie Chloroquin, Mefloquin, Quinin sind gleichermassen wirksame Agentien, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3, Tubeimosid, Bruceanole A, B, C, Bruceantinoside C, Yadanzioside N, und P, Isodeoxyelephantopin, Tomenphantopin A und B, Coronarin A, B, C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, 1,11-Dimethoxycanthin-6-on, 1-Hydroxy-11-Methoxycanthin-6-on, Scopolectin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien-4,16-dien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychno-pentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B, weitere natürliche Terpenoide wie Hippocaesculin, 14-Dehydroagrostistachin, C-Type Natriuretic Peptide (CNP), Agroskerin, Agrostistachin, 17-Hydroxyagrostistachin, Ovatodiolide, 4,7-Oxy-cycloanisomelsäure, Yadanzioside N und P, Isodeoxyelephantopin, Tomenphantopin A und B, Coronarin A, B, C und D, Ursolsäure, Hyptatsäure A, Zeorin, Iso-Iridogermanal, Maytenfoliol, Effusantin A, Excisanin A und B, Longikaurin B, Sculponeatin.

Der oder die Wirkstoffe können dabei als solche allein, aber auch eingebettet in eine bioresorbierbare Trägersubstanz, wie zum Beispiel das Polymermaterial des Stentgrundkörpers 2 vorliegen. Auch eine Mikroverkapselung des Wirkstoffes ist möglich.

Auf seiner Außenseite ist der Stentgrundkörper 2 mit einer wirkstoffabgebenden Schicht, einer sogenannten "drug eluting"-Schicht 5 belegt, die beispielsweise aus einer anti-proliferativ wirkenden Substanz eingebettet wiederum in ein Trägermaterial gebildet sein kann.

Aufgrund der Anordnung der Wirkstoffdepots 4 als Seele 3 in dem Stentgrundkörper 2 werden die Wirkstoffe in dem Wirkstoffdepot 4 genau dann freigesetzt, wenn der Stentgrundkörper 2 soweit einen Abbau seiner Polymerketten vollzogen hat, dass ein Massenabbau des Polymermaterials erfolgt. Insoweit ist der die Umhüllung des Wirkstoffdepots 4 bildende Stentgrundkörper eine zeitlich veränderliche Umhüllung, die so ausgelegt ist, dass die Freisetzung des Wirkstoffs aus dem Wirkstoffdepot 4 auf den Massenabbau des biodegradierbaren Polymermaterials der Stentstreben 1 zeitlich abgestimmt erfolgt.

Die in Fig. 2 gezeigte Ausführungsform unterscheidet sich von der gemäß Fig. 1 lediglich darin, dass die Seele 3 von einer Trennschicht 6 von dem das Wirkstoffdepot 4 umhüllenden Stentgrundkörper 2 separiert ist. Diese Trennschicht 6 dient also einer makroskopischen Verkapselung des Wirkstoffdepots 4 und dient als Diffusionsbremse für den Wirkstoff. Dadurch kann - soweit dies für die auf den Massenabbau des Stentstrebenmaterials zeitlich abgestimmte Freisetzung des Wirkstoffes erforderlich ist - ein zeitlich verzögerter Start der Wirkstofffreisetzung gesteuert werden. Die Trennschicht besteht typischerweise aus einem degradablen Polymer wie z.B. Polydioxanon, Polyglycolid Polycaprolacton, Polylactide (Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(1-Lactid-co-trimethylen carbonat, Triblockcopolymere), Polysaccharide (Chitosan, Levan, Hyaluronsäure, Heparin, Dextran, Cellulose etc.), Polyhydroxyvalerat, Ethylvinylacetat, Polyethylenoxid, Polyphosphorylcholin, Fibrin oder Albumin.
Es ist auch möglich, dass die Trennschicht permeabel ist und aus einem permanenten Polymer wie z.B. Parylene, Polypropylen, Polyethylen, Polyvinylchlorid, Polymethylmethacrylat, Polymethylmethacrylat, Polytetrafluorethylen, Polyvinylalkohol, Polyurethan, Polybuthylenterephthalat, Silikon, Polyphosphazen sowie deren Copolymeren und Blends oder aus anorganischen Schichten besteht. Im Fall einer nicht degradablen Trennschicht ist die Schichtdicke so zu wählen, vorzugsweise zwischen ungefähr 0.2 µm bis ungefähr 5µm, dass der gekapselte Wirkstoff noch mit der richtigen Freisetzungskinetik abgegeben wird. Für den verbleibenden Aufbau der Stentstrebe 1 gemäß Fig. 2 kann auf die Ausführungen zu Fig. 1 verwiesen werden. Übereinstimmende Elemente sind mit identischen Bezugszeichen versehen.

Beim Ausführungsbeispiel gemäß Fig. 3 ist das Wirkstoffdepot 4 als Zwischenschicht 7 ausgelegt, die zwischen zwei Lagen 8, 9 des Stentgrundkörpers 2 eingebettet ist. Bei dieser Auslegung des Wirkstoffdepots 4 ist es von Vorteil, wenn der Wirkstoff in der Zwischenschicht 7 in einen Träger mit langsamer Diffusion eingebettet ist.

Auch der in Fig. 3 gezeigte Stentgrundkörper 2 weist eine Außenschicht 5 als Drug-eluting-Schicht auf.

In den Fig. 4 und 5 schließlich ist eine Ausführungsform eines Stentgrundkörpers 2 gezeigt, bei der die Wirkstoffdepots 4 in Vertiefungen 10 (siehe linker Teil der Fig. 5) bzw. Durchbrüchen 11 (siehe rechter Teil der Fig. 5) angelegt sind. Die offenen Seiten dieser Vertiefungen 10 bzw. Durchbrüche 11 mit den offenliegenden Flächen der Wirkstoffdepots 4 sind dabei durch eine nicht oder nur sehr langsam degradierende Sperrschicht (12) mit hoher Barrierewirkung verschlossen (z.B. Parylen, BUMA, PLLA). Damit bildet wiederum der Stentgrundkörper 2 selbst die sich zeitlich verändernde Umhüllung der Wirkstoffdepots 4, die mit dem Massenabbau des Stent-Polymermaterials geöffnet und damit der Wirkstoff seine therapeutische Wirkung entfalten können. Der Wirkstoff in diesen Wirkstoffdepots 4 in den Vertiefungen 10 bzw. Durchbrüchen 11 ist wiederum in einen Träger eingebettet.

Die Herstellung der in den Fig. 1 bis 5 gezeigten Stentstreben 1 mit Wirkstoffdepots 4 kann durch übliche Fertigungsverfahren, wie eine Kombination aus Spanen, Bohren und Laserschneiden für den Abtrag von Material zur Bildung des Stentgrundkörpers 2, Tauchen und Sprühen für den Auftrag von Schichten sowie Gießen in eine Hohlform für das Erstellen ausgedehnter Körper, wie Zylinder, eingesetzt werden.

In Fig. 1 lässt sich die Stentgrundkörper 2 zum Beispiel für den Fall eines geflochtenen Stents (sog. Wallstent-Design) klassisch extrudieren. Im zweiten Schritt wird das Wirkstoffdepot 4 mit einem Wirkstoff-Lösungsmittelgemisch (ggf. mit einer zusätzlichen Trägersubstanz) ausgegossen, und das Lösungsmittel wird ggf. durch Temperaturerhöhung ausgetrieben. Dieser Vorgang kann wiederholt werden, um entstandene Hohlräume auszufüllen. Alternativ zu Schritt 2 ist auch eine Befüllung mit einem pulverartigen Wirkstoff möglich. Die Drug-eluting-Schicht 5 kann zum Beispiel durch ein traditionelles Sprühverfahren mit Lösungsmittel als letzter Schritt aufgebracht werden.

Alternativ lässt sich auch eine Seele 3 in flüssiger Form in eine Hohlform gießen, die sich öffnen lässt. Nach Abkühlen wird die Seele in eine größere Hohlform eingebracht, und der Stentgrundkörper 2 wird eingebracht, ggf. eingepresst.

Die Trennschicht 6 in Fig. 2 lässt sich als Zwischenschicht einfügen, indem die Seele 3 mittels Sprühverfahren beschichtet wird, bevor er mit dem Stentgrundkörper 2 ummantelt wird.

Ein Stent gemäß Fig. 3 lässt sich aus lasergeschnittenen Schichten zusammensetzen oder aber aus einem Sandwichrohr mittels Laserschneiden herauspräparieren.

Die Durchbrüche 11 bzw. Vertiefungen 10 gemäß Fig. 4 lassen sich durch Laserablation in Vollmaterial einbringen. Die Sperrschichten 12 an diesen Vertiefungen 10 oder Durchbrüchen 11 lassen sich z.B. mit einer Pipettiertechnik schrittweise aufbringen.

## Patentansprüche

1. Stent umfassend
- Stentstreben (1) aus einem biodegradierbaren Polymermaterial, und
- Wirkstoffdepots (4) mit mindestens einem Wirkstoff in den Stentstreben (1), **gekennzeichnet durch**
- mindestens einen die Abbaufolgen der Stentstreben (1) therapierenden Wirkstoff in den Wirkstoffdepots (4), und
- eine zeitlich veränderliche Umhüllung der Wirkstoffdepots (4) derart, dass die Freisetzung des Wirkstoffs aus den Wirkstoffdepots (4) auf den Massenabbau des biodegradierbaren Polymermaterials der Stentstreben (1) zeitlich abgestimmt erfolgt.

2. Stent nach Anspruch 1, **dadurch gekennzeichnet, dass** der Wirkstoff ein Inflammationshemmer und/oder heilungsfördernder Wirkstoff ist.

3. Stent nach Anspruch 1 oder 2, **dadurch gekennzeichnet, dass** zur Bildung der Umhüllung die Wirkstoffdepots (4) als Seele (3) in den Stentstreben (1) angelegt sind.

4. Stent nach Anspruch 3, **dadurch gekennzeichnet, dass** die Seele (3) durch eine abbaubare oder permeable Trennschicht (6) von der sie umhüllenden Stentstrebe (1) separiert ist.

5. Stent nach Anspruch 1 oder 2, **dadurch gekennzeichnet, dass** zur Bildung der Umhüllung die Wirkstoffdepots (4) als Zwischenschicht (7) in den Stentstreben (1) angelegt sind.

6. Stent nach Anspruch 1 oder 2, **dadurch gekennzeichnet, dass** zur Bildung der Umhüllung die Wirkstoffdepots (4) in Vertiefungen (10) oder Durchbrüchen (11) der Stentstreben (1) angelegt sind, wobei ihre offenen Seiten durch eine nicht oder nur langsam abbaubare Sperrschicht (12) verschlossen sind.

7. Stent nach einem der vorgenannten Ansprüche, **dadurch gekennzeichnet, dass** jeder Wirkstoff allein, eingemischt in ein Polymerträgermaterial oder mikroverkapselt vorliegt.

8. Stent nach einem der vorgenannten Ansprüche, **dadurch gekennzeichnet, dass** die Stentstreben (1) auf ihrer Außenseite mit einer Wirkstoff-abgebenden Außenschicht (5) versehen sind.

9. Stent nach Anspruch 8, **dadurch gekennzeichnet, dass** die Außenschicht (5) eine anti-proliferativen wirkende Substanz eingebettet in einen Träger aufweist.
